# EUROPEAN PATENT APPLICATION

(11) **EP 0 891 986 A1**
(43) Date of publication of application: **20.01.1999**
(21) Application number: 97810486.7
(22) Date of filing: 15.07.1997
(51) Int. Cl.: C08F 2/00, C08F 2/38, C08F 4/00, C07C 239/20

(54) **Polymerizable compositions containing alkoxyamine compounds derived from nitrogen oxide**

(71) Applicant: Ciba Specialty Chemicals Holding Inc., 4057 Basel (CH)
(72) Inventor: Nesvadba, Peter, 1723 Marly (CH); Kramer, Andreas, 3186 Düdingen (CH); Steinmann, Alfred, 1724 Praroman (CH); Stauffer, Werner, 1700 Fribourg (CH)

(57) **Abstract**

A polymerizable composition, comprising
a) at least one ethylenically unsaturated monomer or oligomer, and
b) an initiator compound of formula (I) 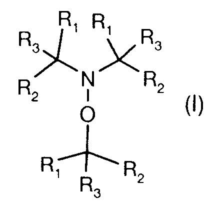
wherein R₁, R₂, R₃ are each independently of one another hydrogen, halogen, NO₂, cyano, -CONR₅R₆, -(R₉)COOR4, -C(O)-R₇, -OR₈, -SR₈, -NHR₈, -N(R₈)₂, carbamoyl, di(C₁-C₁₈alkyl)carbamoyl, -C(=NR₅)(NHR₆);
unsubstituted C₁-C₁₈alkyl, C₂-C₁₈alkenyl, C₂-C₁₈alkynyl, C₇-C₉phenylalkyl, C₃-C₁₂cycloalkyl or C₂-C₁₂heterocycloalkyl; or
C₁-C₁₈alkyl, C₂-C₁₈alkenyl, C₂-C₁₈ alkynyl, C₇-C₉phenylalkyl, C₃-C₁₂cycloalkyl or C₂-C₁₂heterocycloalkyl, which are substituted by NO₂, halogen, amino, hydroxy, cyano, carboxy, C₁-C₄alkoxy, C₁-C₄alkylthio, C₁-C₄alkylamino or di(C₁-C₄alkyl)amino; or
phenyl, naphthyl, phenanthryl, anthryl, pyrrolyl, imidazolyl, pyrazolyl, pyrazinyl, pyrimidinyl, pyridazinyl, indolizinyl, isoindolyl, indolyl, indazolyl, purinyl, quinolizinyl, isoquinolyl, quinolyl, quinoxalinyl, quinazolinyl, cinnolinyl, pteridinyl, phenazinyl, isothiazolyl, phenothiazinyl, isoxazolyl, furazanyl, biphenyle or phenoxazinyl, which are unsubstituted or substituted by C₁-C₄alkyl, C₁-C₄alkoxy, C₁-C₄alkylthio, halogen, cyano, hydroxy, carboxy, C₁-C₄alkylamino or di(C₁-C₄alkyl)amino;
or R₂ and R₃, together with the linking carbon atom, form a C₃-C₁₂ cycloalkyl radical, a (C₄-C₁₂cycloalkanon)-yl radical or a C₂-C₁₂heterocycloalkyl radical containing at least one O atom and/or a NR₈ group;
R₄ is hydrogen, C₁-C₁₈alkyl, phenyl, an alkali metal cation or a tetraalkylammonium cation;
R₅ and R₆ are hydrogen, C₁-C₁₈alkyl, C₂-C₁₈alkyl which is substituted by at least one hydroxy group or, taken together, form a C₂-C₁₂alkylene bridge or a C₂-C₁₂-alkylene bridge interrupted by at least one O or/and NR₈ atom;
R₇ is hydrogen, C₁-C₁₈alkyl or phenyl;
R₈ is hydrogen, C₁-C₁₈alkyl or C₂-C₁₈alkyl which is substituted by at least one hydroxy group;
R₉ is C₁-C₁₂alkylen or a direct bond;
or R₁, R₂ and R₃ form together the residue of a polycyclic cycloaliphatic ring system or a polycyclic heterocycloaliphatic ring system with at least one di- or trivalent nitrogen atom. Further aspects of the present invention are a process for polymerizing ethylenically unsaturated monomers, novel initiator compounds and their use for polymerization, and also the polymer or copolymer produced by this process.

## Description

The present invention relates to a polymerizable composition comprising a) at least one ethylenically unsaturated monomer and b) a nitroxide initiator compound. Further aspects of the present invention are a process for polymerizing ethylenically unsaturated monomers, novel initiator compounds and their use for polymerization, and also the polymer or copolymer produced by this process.

More specifically, in one of its aspects the present invention relates to polymerizable compositions and polymerization processes which provide polymeric resin products having low polydispersity , which polymerization processes proceed with enhanced monomer to polymer conversion efficiencies. In particular, this invention relates to stable free radical-mediated polymerization processes which provide homopolymers, random copolymers, block copolymers, multiblock copolymers, graft copolymers and the like, at enhanced rates of polymerization and enhanced monomer to polymer conversions.

Polymers or copolymers prepared by free radical polymerization processes inherently have broad molecular weight distributions or polydispersities which are generally higher than about four. One reason for this is that most of the free radical initiators have half lives that are relatively long, ranging from several minutes to many hours, and thus the polymeric chains are not all initiated at the same time and the initiators provide growing chains of various lengths at any time during the polymerization process. Another reason is that the propagating chains in a free radical process can react with each other in processes known as combination and disproportionation, both of which are irreversibly chain-terminating reaction processes. In doing so, chains of varying lengths are terminated at different times during the reaction process, resulting in resins consisting of polymeric chains which vary widely in length from very small to very large and which thus have broad polydispersities. If a free radical polymerization process is to be used for producing narrow molecular weight distributions, then all polymer chains must be initiated at about the same time and termination of the growing polymer-chains by combination or disproportionation processes must be avoided.

Conventional radical polymerization reaction processes pose various significant problems, such as difficulties in predicting or controlling the molecular weight, the polydispersity and the modality of the polymers produced. These prior art polymerization processes produce polymers having broad polydispersities and in some instances, low polymerization rates. Furthermore, free radical polymerization processes in bulk of the prior art are difficult to control because the polymerization reaction is strongly exothermic and an efficient heat removal in the highly viscous polymer is mostly impossible. The exothermic nature of the prior art free radical polymerization processes often severely restricts the concentration of reactants or the reactor size upon scale-up.

Due to the above mentioned uncontrollable polymerization reactions, gel formation in conventional free radical polymerization processes are also possible and cause broad molecular weight distributions and/or difficulties during filtering, drying and manipulating the product resin.

US-A-4 581 429 to Solomon et al., issued April 8, 1986, discloses a free radical polymerization process which controls the growth of polymer chains to produce short chain or oligomeric homopolymers and copolymers, including block and graft copolymers. The process employs an initiator having the formula (in part) R'R''N-O-X, where X is a free radical species capable of polymerizing unsaturated monomers. The reactions typically have low conversion rates. Specifically mentioned R'R''N-O· radical groups are derived from tetraethylisoindoline, tetrapropylisoindoline, tetramethylpiperidine, tetramethylpyrrolidine or di-t-butylamine.

Symmetrically substituted initiators where R', R'' and X are the same groups are not mentioned.

EP-A-735 052 discloses a method of preparing thermoplastic polymers of narrow polydispersities by free radical-initated polymerization, which comprises adding a free radical initiator and a stable free radical agent to the monomer compound.

This method has the disadvantage that uncontrollable recombinations of initiator radicals occur immidiately after their formation, thus producing variable ratios between initiator radicals and stable free radicals. Consequently there is no control about the polymerization process.

There is therefore still a need for polymerization processes for the preparation of narrow polydispersity polymeric resins with defined molecular weights using the economical free radical polymerization techniques. These polymerization processes will also control the physical properties of the polymers such as viscosity, hardness, gel content, processability, clarity, high gloss, durability, and the like.

The polymerization processes and resin products of the present invention are useful in many applications, including a variety of specialty applications, such as for the preparation of block copolymers which are useful as compatibilizing agents for polymer blends, or dispersing agents for coating systems or for the preparation of narrow molecular weight resins or oligomers for use in coating technologies and thermoplastic films or as toner resins and liquid immersion development ink resins or ink additives used for electrophotographic imaging processes.

Surprisingly, it has now been found that it is possible to overcome the afore mentioned shortcomings of the prior art by providing a polymerizable composition containing specific initiator compounds. The majority of these compounds are novel and they are also an object of the present invention. Polymerization of the composition results in a polymer or copolymer of narrow polydispersity and a high monomer to polymer conversion even at relative low temperatures and at short reaction times, making the polymerization process particularly suitable for industrial applications. The resulting copolymers are of high purity and in many cases colourless, therefore not requiring any further purification.

One object of the present invention is to provide a polymerizable composition, comprising
a) at least one ethylenically unsaturated monomer or oligomer, and
b) an initiator compound of formula (I) 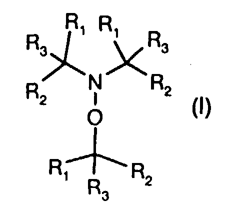
wherein R₁, R₂, R₃ are each independently of one another hydrogen, halogen, NO₂, cyano, -CONR₅R₆, -(R₉)COOR4, -C(O)-R₇, -OR₈, -SR₈, -NHR₈, -N(R₈)₂, carbamoyl, di(C₁-C₁₈alkyl)carbamoyl, -C(=NR₅)(NHR₆);
unsubstituted C₁₋₁₈alkyl, C₂-C₁₈alkenyl, C₂₋₁₈alkynyl, C₇₋C₉phenylalkyl, C₃₋C₁₂cycloalkyl or C₂-C₁₂heterocycloalkyl; or
C₁₋₁₈alkyl, C₂₋C₁₈alkenyl, C₂₋C₁₈ alkynyl, C₇₋C₉phenylalkyl, C₃₋C₁₂cycloalkyl or C₂₋ C₁₂heterocycloalkyl, which are substituted by NO₂, halogen, amino, hydroxy, cyano, carboxy, C₁₋C₄alkoxy, C₁₋C₄alkylthio, C₁₋C₄alkylamino or di(C₁₋C₄alkyl)amino; or
phenyl, naphthyl, phenanthryl, anthryl, pyrrolyl, imidazolyl, pyrazolyl, pyrazinyl, pyrimidinyl, pyridazinyl, indolizinyl, isoindolyl, indolyl, indazolyl, purinyl, quinolizinyl, isoquinolyl, quinolyl, quinoxalinyl, quinazolinyl, cinnolinyl, pteridinyl, phenazinyl, isothiazolyl, phenothiazinyl, isoxazolyl, furazanyl, biphenyle or phenoxazinyl, which are unsubstituted or substituted by C₁₋C₄alkyl, C₁₋C₄alkoxy, C₁₋C₄alkylthio, halogen, cyano, hydroxy, carboxy, C₁-C₄alkylamino or di(C₁₋C₄alkyl)amino;
or R₂ and R₃, together with the linking carbon atom, form a C₃₋C₁₂ cycloalkyl radical, a (C₄-C₁₂cycloalkanon)-yl radical or a C₂₋C₁₂heterocycloalkyl radical containing at least one O atom and/or a NR₈ group;
R₄ is hydrogen, C₁-C₁₈alkyl, phenyl, an alkali metal cation or a tetraalkylammonium cation;
R₅ and R₆ are hydrogen, C₁-C₁₈alkyl, C₂₋C₁₈alkyl which is substituted by at least one hydroxy group or, taken together, form a C₂₋C₁₂alkylene bridge or a C₂₋C₁₂-alkylene bridge interrupted by at least one O or/and NR₈ atom;
R₇ is hydrogen, C₁-C₁₈alkyl or phenyl;
R₈ is hydrogen, C₁-C₁₈alkyl or C₂-C₁₈alkyl which is substituted by at least one hydroxy group; R₉ is C₁₋C₁₂alkylen or a direct bond;
or R₁, R₂ and R₃ form together the residue of a polycyclic cycloaliphatic ring system or a polycyclic heterocycloaliphatic ring system with at least one di- or trivalent nitrogen atom.

The initiator compound of formula (I) is preferably present in an amount of 0.01 mol-% to 100 mol-%, more preferably in an amount of 0.1 mol-% to 10 mol-%, based on the monomer, oligomer or monomer/oligomer mixture used.

Halogen is fluoro, chloro, bromo or iodo.

The alkyl radicals in the various substituents may be linear or branched. Examples of alkyl containing 1 to 18 carbon atoms are methyl, ethyl, propyl, isopropyl, butyl, 2-butyl, isobutyl, t-butyl, pentyl, 2-pentyl, hexyl, heptyl, octyl, 2-ethylhexyl, t-octyl, nonyl, decyl, undecyl, dodecyl, tridecyl, tetradecyl, hexadecyl and octadecyl.

The alkenyl radicals in the various substituents may be linear or branched. Examples of C₂-C₁₈alkenyl are vinyl, ally, 2-methylallyl, butenyl, hexenyl, undecenyl and octadecenyl. Preferred alkenyls are those, wherein the carbon atom in the 1-position is saturated and where the double bond is not activated by substituents like O, C=O, and the like.

Examples of C₂-C₁₈alkynyl are ethynyl,2-butynyl, 3-hexynyl, 5-undecynyl, 6-octadecynyl. The alkynyl radicals may be linear or branched.

C₇₋C₉phenylalkyl is for example benzyl, phenylpropyl, a,a-dimethylbenzyl or a-methylbenzyl.

C₃₋C₁₂cycloalkyl which is unsubstituted or substituted by 1, 2 or 3 C₁₋C₄alkyl is typically cyclopropyl, cyclopentyl, methylcyclopentyl, dimethylcyclopentyl, cyclohexyl, methylcyclohexyl.

Alkyl substituted by-OH is typically 2-hydroxyethyl, 2-hydroxypropyl or 2-hydroxybutyl.

C₁₋₁₈Alkyl substituted by C₁₋C₁₈alkoxy, preferably by C₁₋C₄alkoxy, in particular by methoxy or ethoxy, is typically 2-methoxyethyl, 2-ethoxyethyl, 3-methoxypropyl, 3-ethoxypropyl, 3-butoxypropyl, 3-octoxypropyl and 4-methoxybutyl.

C₁-C₁₈Alkyl substituted by di(C₁-C₄alkyl)amino is preferably e.g. dimethylamino, diethylamino, 2-dimethylaminoethyl, 2-diethylaminoethyl, 3-dimethylaminopropyl, 3-diethylaminopropyl, 3-dibutylaminopropyl and 4-diethylaminobutyl.

C₁-C₁₈alkyl substituted by C₁-C₄alkylamino is preferably e.g. methylamino, ethylamino, 2-methylaminoethyl, 2-ethylaminoethyl, 3-methylaminopropyl, 3-ethylaminopropyl, 3-butylaminopropyl and 4-ethylaminobutyl.

C₁-C₈alkoxy and, preferably C₁-C₄alkoxy, are typically methoxy, ethoxy, propoxy, isopropoxy, butoxy, isobutoxy, pentoxy, isopentoxy, hexoxy, heptoxy or octoxy.

C₁-C₄Alkylthio is typically thiomethyl, thioethyl, thiopropyl, thioisopropyl, thiobutyl and thioisobutyl.

C₂-C₁₂heterocycloalkyl is typically oxirane, 1,4-dioxane, tetrahydrofuran, g-butyrolactone, e-caprolactam, oxirane, aziridine, diaziridine, pyrrole, pyrrolidine, thiophen, furan, pyrazole, imidazole, oxazole, oxazolidine, thiazole, pyran, thiopyran, piperidine or morpholine.

Examples of C₂-C₁₂alkylene bridges, preferably of C₂-C₆alkylene bridges, are ethylene, propylene, butylene, pentylene, hexylene.

C₂-C₁₂Alkylene bridges interrupted by at least one N or O atom are, for example, -CH₂-O-CH₂-CH₂, -CH₂-O-CH₂-CH₂-CH₂, -CH₂-O-CH₂-CH₂-CH₂-CH₂-, -CH₂-O-CH₂-CH₂-O-CH2-, -CH₂-NH-CH₂-CH₂, -CH₂-NH-CH₂-CH₂-CH₂, -CH₂-NH-CH₂-CH₂-CH₂-CH₂-, -CH₂-NH-CH₂-CH₂-NH-CH2- or -CH₂-NH-CH₂-CH₂-O-CH2-.

Examples for C₄-C₁₂cycloalkanone-yl are cyclopentanone-yl, cyclohexanone-yl or cycloheptanone-yl.

Phenyl substituted by 1,2 or 3 C₁-C₄alkyl or C₁-C₄alkoxy is typically methylphenyl, dimethylphenyl, trimethylphenyl, t-butylphenyl, di-t-butylphenyl, 3,5-di-t-butyl-4-methylphenyl, methoxyphenyl, ethoxyphenyl and butoxyphenyl.

Examples of polycyclic cycloaliphatic ring systems are adamantane, cubane, twistane, norbornane, bycyclo[2.2.2]octane or bycyclo[3.2.1]octane.

An example of a polycyclic heterocycloaliphatic ring system is hexamethylentetramine (urotropine).

The C-atom to which the substituents R₁, R₂ and R₃ are bound is preferably a secondary or tertiary C-atom more preferably it is a tertiary C-atom.

The monomers suitable for use in the present invention may be water-soluble or water-insoluble. Water soluble monomers contain typically a carboxylic acid group or a salt of a carboxylic acid group. Water insoluble monomers are typically free of acid and phenolic groups.

Typical monoethylenically unsaturated monomers free of carboxylic acid and phenolic groups which are suitable for this invention include the alkyl esters of acrylic or methacrylic acids such as methyl acrylate, ethyl acrylate, butyl acrylate, methyl methacrylate, ethyl methacrylate, butyl methacrylate and isobutyl methacrylate; the hydroxyalkyl esters of acrylic or methacrylic acids, such as hydroxyethyl acrylate, hydroxypropyl acrylate, hydroxyethyl methacrylate, and hydroxypropyl methacrylate; acrylamide, methacrylamide, N-tertiary butylacrylamide, N-methylacrylamide, N,N-dimethylacrylamide; acrylonitrile, methacrylonitrile, ally alcohol, dimethylaminoethyl acrylate, dimethylaminoethyl methacrylate, phosphoethyl methacrylate, N-vinylpyrrolidone, N-vinylformamide, N-vinylimidazole, vinyl acetate, conjugated dienes such as butadiene or isoprene, styrene, styrenesulfonic acid salts, vinylsulfonic acid salts and 2-acrylamido-2-methylpropane-sulfonic acid salts and acryloil chloride.

Preferred ethylenically unsaturated monomers or oligomers are selected from the group consisting of styrene, substituted styrene, conjugated dienes, acrolein, vinyl acetate, (alkyl)acrylic acidanhydrides, (alkyl)acrylic acid salts, (alkyl)acrylic esters or (alkyl)acrylamides.

Particularly preferred ethylenically unsaturated monomers are styrene, α methyl styrene, p-methyl styrene or a compound of formula CH₂=C(Rₐ)-(C=Z)-R_{b}, wherein Rₐ is hydrogen or C₁-C₄alkyl, R_{b} is NH₂, O(Me) , glycidyl, unsubstituted C₁₋₁₈alkoxy or hydroxy-substituted C₁-C₁₈alkoxy, unsubstituted C₁-C₁₈alkylamino, di(C₁₋C₁₈alkyl)amino, hydroxy-substituted C₁-C₁₈alkylamino or hydroxy-substituted di(C₁-C₁₈alkyl)amino;
Me is a monvalent metal atom; and
Z is oxygen or sulfur.

Typical metal atoms are Na, K or Li.

Examples and preferences for alkyl, alkoxy, alkylamino, dialkylamino and hydroxy-substituted alkoxy are afore mentioned.

In a particular preferred composition Rₐ is hydrogen or methyl, R_{b} is NH₂, glycidyl, unsubstituted or with hydroxy substituted C₁₋C₄alkoxy, unsubstituted C₁₋C₄alkylamino, di(C₁-C₄alkyl)amino, hydroxy-substituted C₁₋C₄alkylamino or hydroxy-substituted di(C₁-C₄alkyl)amino;and
Z is oxygen.

Most preferred is a polymerizable composition, wherein the ethylenically unsaturated monomer is methylacrylate, ethylacrylate, butylacrylate, isobutylacrylate, tert. butylacrylate, hydroxyethylacrylate, hydroxypropylacrylate, dimethylaminoethylacrylate, glycidylacrylates, methyl(meth)acrylate, ethyl(meth)acrylate, butyl(meth)acrylate, hydroxyethyl(meth)acrylate, hydroxypropyl(meth)acrylate, dimethylaminoethyl(meth)acrylate, glycidyl(meth)acrylates, acrylonitrile, acrylamide or methacrylamide.

Examples of comonomers suitable for use in the present invention are C₃₋C₆ethylenically unsaturated monocarboxylic acids as well as the alkali metal salts and ammonium salts thereof. The C₃-C₆ethylenically unsaturated monocarboxylic acids include acrylic acid, methacrylic acid, crotonic acid, vinylacetic acid and acryloxypropionic acid. Acrylic acid and methacrylic acid are the preferred monoethylenically unsaturated monocarboxylic acid monomers.

Examples for C₈-C₁₆ ethylenically unsaturated phenolics, which may also be used as comonomers include 4-hydroxy styrene, 4-hydroxy, α-methyl styrene, and 2,6-ditert. butyl, 4-vinyl phenol.

Another class of carboxylic acid monomers suitable for use as comonomers in this invention are C₄-C₆-ethylenically unsaturated dicarboxylic acids and the alkali metal and ammonium salts thereof as well as the anhydrides of the cis-dicarboxylic acids. Suitable examples include maleic acid, maleic anhydride, itaconic acid, mesaconic acid, fumaric acid and citraconic acid. Maleic anhydride (and itaconic acid are) is the preferred monoethylenically unsaturated dicarboxylic acid monomer(s).

The acid monomers suitable for use in this invention may be in their acid forms or in the form of the alkali metal salts or ammonium salts of the acid. Suitable bases useful for neutralizing the monomer acids include sodium hydroxide, ammonium hydroxide, potassium hydroxide, and the like. The acid monomers may be neutralized to a level of from 0 to 50% and, preferably, from 0 to about 20%. In many cases, the carboxylic acid monomers may be used in the completely neutralized form. The monomers may be neutralized prior to or during polymerization.
Preferred are neutralized carboxylic acid monomers or anhydrides.

The polymerizable composition of the present invention may additionally comprise a solvent selected from the group consisting of water, alcohols, esters, ethers, ketones, amides, sulfoxides, hydrocarbons and halogenated hydrocarbons.
Preferred initiator compounds are those, wherein R₁, R₂, R₃ are each independently of one another NO₂, cyano, -(R₉)COOR₄, -CONR₅R₆, -C(O)-R₇, -OR₈, carbamoyl, di(C₁-C₁₈alkyl)carbamoyl, -C(=NR₅)(NHR₆);
unsubstituted C₁-C₁₈alkyl, C₇-C₉phenylalkyl, C₃-C₁₂cycloalkyl or C₂-C₁₂heterocycloalkyl; or C₁-C₁₈alkyl, C₇-C₉phenylalkyl, C₃-C₁₂cycloalkyl or C₂-C₁₂heterocycloalkyl, which are substituted by amino, hydroxy, cyano, carboxy, C₁-C₄alkoxy, C₁-C₄alkylamino or di(C₁-C₄alkyl)amino; or
phenyl, naphthyl, phenanthryl, anthryl, pyrrolyl, imidazolyl, pyrazolyl, pyrazinyl, pyrimidinyl, pyridazinyl, indolizinyl, isoindolyl, indolyl, indazolyl, purinyl, quinolizinyl, isoquinolyl, quinolyl, quinoxalinyl, quinazolinyl, cinnolinyl, pteridinyl, phenazinyl, isothiazolyl, phenothiazinyl, isoxazolyl, furazanyl, biphenyle or phenoxazinyl, which are unsubstituted or substituted by C₁-C₄alkyl, C₁-C₄alkoxy, C₁-C₄alkylthio, halogen, cyano, hydroxy, carboxy, C₁-C₄alkylamino or di(C₁-C₄alkyl)amino;
or R₂ and R₃, together with the linking carbon atom, form a C₃-C₁₂ cycloalkyl radical;
R is, C₁-C₁₈alkyl, phenyl, an alkali metal cation or a tetraalkylammonium cation;
R₅ and R₆ are hydrogen, C₁-C₁₈alkyl, C₂-C₁₈alkyl which is substituted by at least one hydroxy group or, taken together, form a C₂-C₁₂alkylene bridge;
R₇ is hydrogen, C₁-C₁₈alkyl or phenyl;
R₈ is C₁-C₁₈alkyl or C₂-C₁₈alkyl which is substituted by at least one hydroxy group; R₉ is C₁-C₄alkylen or a direct bond;
or R₁, R₂ and R₃ form together the residue of a polycyclic cycloaliphatic ring system or a polycyclic heterocycloaliphatic ring system with at least one di- or trivalent nitrogen atom.

More preferred are initiators, wherein R₁, R₂, R₃ are each independently of one another NO₂, cyano, -(R₉)COOR₄, -CONR₅R₆, -C(O)-R₇, OR₈, carbamoyl, di(C₁-C₈alkyl)carbamoyl, - C(=NR₅)(NHR₆);
unsubstituted C₁-C₈alkyl, C₇-C₉phenylalkyl, C₅-C₇cycloalkyl or C₄-C₇heterocycloalkyl; or
C₁-C₈alkyl, C₇-C₉phenylalkyl, C₅-C₇cycloalkyl or C₄-C₇heterocycloalkyl, which are substituted by amino, hydroxy, cyano, carboxy, C₁-C₄alkoxy, C₁-C₄alkylamino or di(C₁-C₄alkyl)amino;
or phenyl, naphthyl, pyrrolyl, imidazolyl, pyrazolyl, pyrazinyl, pyrimidinyl, pyridazinyl, which are unsubstituted or substituted by C₁-C₄alkyl, C₁-C₄alkoxy, C₁-C₄alkylthio, halogen, cyano, hydroxy, carboxy, C₁-C₄alkylamino or di(C₁-C₄alkyl)amino;
or R₂ and R₃, together with the linking carbon atom, form a C₅-C₇cycloalkyl radical;
R₄ is C₁-C₈alkyl, phenyl, an alkali metal cation or a tetraalkylammonium cation;
R₅ and R₆ are hydrogen, C₁-C₈alkyl, C₂-C₈alkyl which is substituted by at least one hydroxy group or, taken together, form a C₂-C₆alkylene bridge;
R₇ is hydrogen, C₁-C₈alkyl or phenyl;
R₈ is C₁-C₈alkyl or C₂-C₈alkyl which is substituted by at least one hydroxy group;
R₉ is C₁-C₄alkylen or a direct bond;
or R₁, R₂ and R₃ form together the residue of a polycyclic cycloaliphatic ring system or a polycyclic heterocycloaliphatic ring system with at least one di- or trivalent nitrogen atom.

Particularly preferred initiators are those, wherein R₁, R₂, R₃ are each independently of one another NO₂, cyano, -(R₉)COOR₄, -CONR₅R₆, -C(O)-R₇, OR₈, carbamoyl, di(C₁-C₁₈alkyl)carbamoyl, -C(=NR₅)(NHR₆);
unsubstituted C₁-C₈alkyl or C₅-C₇cycloalkyl;
or phenyl, which is unsubstituted or substituted by C₁-C₄alkyl, C₁-C₄alkoxy, cyano, hydroxy, carboxy, C₁-C₄alkylamino or di(C₁-C₄alkyl)amino;
or R₂ and R₃, together with the linking carbon atom, form a C₅-C₇ cycloalkyl radical;
R₄ is C₁-C₈alkyl, phenyl, an alkali metal cation or a tetraalkylammonium cation;
R₅ and R₆ are hydrogen, C₁-C₈alkyl, C₂-C₈alkyl which is substituted by at least one hydroxy group or, taken together, form a C₂-C₆alkylene bridge;
R₇ is, C₁-C₈alkyl or phenyl;
R₈ is C₁-C₈alkyl or C₂-C₈alkyl which is substituted by at least one hydroxy group;
R₉ is C₁-C₄alkylen or a direct bond.

A further particularly preferred subgroup of initiators are those, wherein R₁ is NO₂, cyano, - CONR₅R₆, -(R₉)COOR₄, -C(=NR₅)(NHR₆);
R₂ and R₃ are each independently of one another unsubstituted C₁-C₈alkyl, C₅-C₇cycloalkyl or phenyl;
or R₂ and R₃, together with the linking carbon atom, form a C₅-C₇cycloalkyl radical;
R₄ is, C₁-C₈alkyl, phenyl;
R₅ and R₆ are hydrogen, C₁-C₈alkyl, C₂-C₈alkyl which is substituted by at least one hydroxy group or, taken together, form a C₂-C₆alkylene bridge;
R₉ is C₁-C₄alkylen or a direct bond.

Most preferred are the following initiators, wherein the group 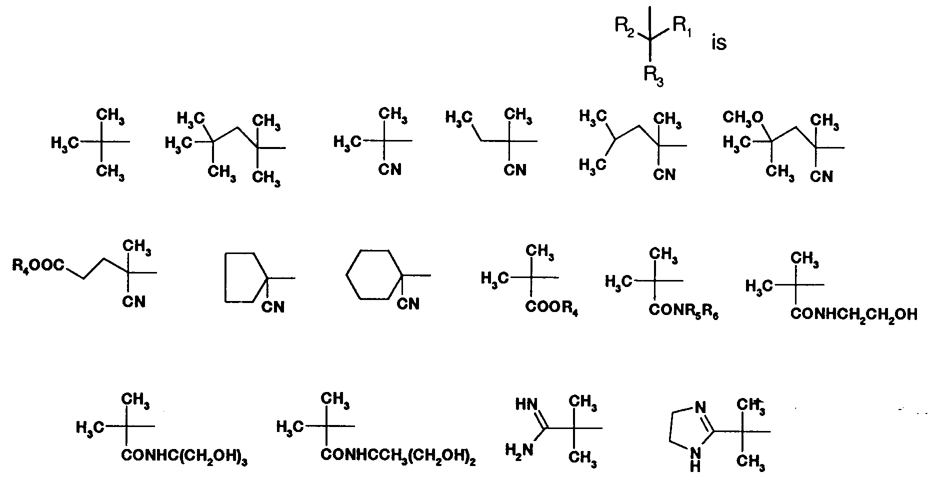 R₁, R₂, R₃, R₄, R₅, R₆, R₇, R₈ and R₉ have the meanings and preferred meanings cited above.

This invention also relates to a free radical polymerization process and polymers obtained thereby, which process overcomes many of the problems and disadvantages of the afore mentioned prior art processes.

This process is used for preparing an oligomer, a cooligomer, a polymer or a copolymer (block or random) by free radical polymerization of at least one ethylenically unsaturated monomer/oligomer, which comprises (co)polymerizing the monomer or monomers/oligomers in the presence of an initiator compound of formula (I) 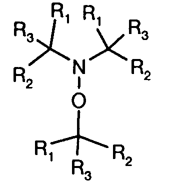 according to claim 1 under reaction conditions capable of effecting scission of the O-C bond to form two free radicals, the radical ·CR₁ R₂R₃ being capable of initiating polymerization.

Preferably, the scission of the O-C bond is effected by heating, ultrasonic treatment, exposure to electromagnetic radiation, ranging from γ to microwaves.

To perform the scission of the O-C bond by heating, the temperature is particularly preferably raised to more than 25°C and less than 150°C.

The process may be carried out in the presence of an organic solvent or in the presence of water or in mixtures of organic solvents and water. Additional cosolvents or surfactants, such as glycols or ammonium salts of fatty acids, may be present. Other suitable cosolvents are described hereinafter.

Preferred processes use as little solvents as possible. In the reaction mixture it is preferred to use more than 30% by weight of monomer and initiator, particularly preferably more than 50% and most preferrably more than 80%.

If organic solvents are used, suitable solvents or mixtures of solvents are typically pure alkanes (hexane, heptane, octane, isooctane), hydrocarbons (benzene, toluene, xylene), halogenated hydrocarbons (chlorobenzene), alkanols (methanol, ethanol, ethylene glycol, ethylene glycol monomethyl ether), esters (ethyl acetate, propyl, butyl or hexyl acetate) and ethers (diethyl ether, dibutyl ether, ethylene glycol dimethyl ether), or mixtures thereof.

The aqueous polymerization reactions can be supplemented with a water-miscible or hydrophilic cosolvent to help ensure that the reaction mixture remains a homogeneous single phase throughout the monomer conversion. Any water-soluble or water-miscible cosolvent may be used, as long as the aqueous solvent medium is effective in providing a solvent system which prevents precipitation or phase separation of the reactants or polymer products until after all polymerization reactions have been completed. Exemplary cosolvents useful in the present invention may be selected from the group consisting of aliphatic alcohols, glycols, ethers, glycol ethers, pyrrolidines, N-alkyl pyrrolidinones, N-alkyl pyrrolidones, polyethylene glycols, polypropylene glycols, amides, carboxylic acids and salts thereof, esters, organosulfides, sulfoxides, sulfones, alcohol derivatives, hydroxyether derivatives such as butyl carbitol or cellosolve, amino alcohols, ketones, and the like, as well as derivatives thereof and mixtures thereof. Specific examples include methanol, ethanol, propanol, dioxane, ethylene glycol, propylene glycol, diethylene glycol, glycerol, dipropylene glycol, tetrahydrofuran, and other water-soluble or water-miscible materials, and mixtures thereof. When mixtures of water and water-soluble or water-miscible organic liquids are selected as the aqueous reaction media, the water to cosolvent weight ratio is typically in the range of about 100:0 to about 10:90.

The initiator compound is preferably present in an amount of 0.01 mol-% to 10 mol-%, more preferably in an amount of 0.1 mol-% to 5 mol-%, based on the monomer or monomer mixture used.

When monomer mixtures or monomer/oligomer mixtures are used, the calculation of mol-% is based on an average molecular weight of the mixture.

Hydrophilic monomers, polymers and copolymers of the present invention can be separated from one another or from the polymerization reaction mixture by, for example, changing the pH of the reaction media and by other well known conventional separation techniques.

The polymerization temperature may range from about 50°C to about 180°C, preferably from about 80°C to about 150°C. At temperatures above about 180°C, the controlled conversion of the monomer into polymer decreases, and uncertain and undesirable by-products like thermally initiated polymer are formed or destruction of the polymerization regulator may occur. Frequently, these by-products discolor the polymer mixture and a purification step may be required to remove them, or they may be intractable.

Therefore the surprisingly high reactivity of the present initiators which are already active at relatively low temperatures leads to short reaction times. The resulting polymers are usually colourless and they can be used in most cases without any further purification step. This is an important advantage when industrial scale-up is considered.

After the polymerizing step is complete, the formed (co)polymer obtained is isolated. The isolating step of the present process is conducted by known procedures, e.g. by distillig off the unreacted monomer or by precipitation in a suitable nonsolvent, filtering the precipitated polymer followed by washing and drying the polymer.

Yet another embodiment of this invention is a method for preparing a block copolymer comprising at least two stages, which comprises forming a polymer with alkoxyamine end groups of the general structure of formula II 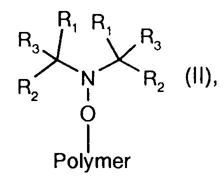 wherein R₁, R₂ and R₃ have the meanings and preferred meanings defined above, the polymer containing the initiator group -CR₁R₂R₃ and having the oxyamine group essentially attached as terminal group, and adding a further monomer followed by heating to form a block copolymer.
Suitable monomers are those mentioned above. The polymer of formula (II) may be isolated prior to the next reaction step or it may be used without isolation, and the second monomer is added to the reaction mixture of the first step.

Block copolymers are, for example, block copolymers of polystyrene and polyacrylate (e.g., Poly(styrene-co-acrylate) or Poly(styrene-co-acrylat-co-styrene). They are usefull as adhesives or as compatibilizers for polymer blends or as polymer toughening agents. Poly(methylmethacrylate-co- acrylate) diblock copolymers or Poly(methylacrylate-co-acrylate-co-methacrylate) triblock copolymers) are useful as dispersing agents for coating systeme, as coating additives (e.g. rheological agents, compatibilizers, reactive diluents) or as resin component in coatings(e.g. high solid paints) Block copolymers of styrene, (meth)acrylates and/or acrylonitrile are useful plastics, elastomers and adhesives.

Furthermore, block copolymers of this invention, wherein the blocks alternate between polar monomers and non-polar monomers, are useful in many applications as amphiphilic surfactants or dispersants for preparing highly uniform polymer blends.

The (co)polymers of the present invention may have a number average molecular weight from 1 000 to 400 000 g/mol, preferably from 2 000 to 250 000 g/mol and, more preferably, from 2 000 to 200 000 g/mol. When produced in bulk, the number average molecular weight may be up to 500 000 (with the same minimum weights as mentioned above). The number average molecular weight may be determined by size exclusion chromatography (SEC), gel permeation chromatography (GPC), matrix assisted laser desorption/ionization mass spectrometry (MALDI-MS) or, if the initiator carries a group which can be easily distinguished from the monomer(s), by NMR spectroscopy or other conventional methods.

Thus, the present invention also encompasses in the synthesis novel block, multi-block, star, gradient, random, hyperbranched and dendritic copolymers, as well as graft or copolymers.

The polymers prepared by the present invention are useful for example in following applications:
adhesives, detergents, dispersants, emulsifiers, surfactants, defoamers, adhesion promoters, corrosion inhibitors, viscosity improvers, lubricants, rheology modifiers, thickeners, crosslinkers, paper treatment, water treatment, electronic materials, paints, coatings, photography, ink materials, imaging materials, superabsorbants, cosmetics, hair products, preservatives, biocide materials or modifiers for asphalt, leather, textiles, ceramics and wood.

Because the present polymerizaton is a "living" polymerization, it can be started and stopped practically at will. Furthermore, the polymer product retains the functional alkoxyamine group (R₁R₂R₃C)₂N-O allowing a continuation of the polymerization in a living matter. Thus, in one embodiment of this invention, once the first monomer is consumed in the initial polymerizing step a second monomer can then be added to form a second block on the growing polymer chain in a second polymerization step. Therefore it is possible to carry out additional polymerizations with the same or different monomer(s) to prepare multi-block copolymers.
Furthermore, since this is a radical polymerization, blocks can be prepared in essentially any order. One is not necessarily restricted to preparing block copolymers where the sequential polymerizing steps must flow from the least stabilized polymer intermediate to the most stabilized polymer intermediate, such as is the case in ionic polymerization. Thus it is possible to prepare a multi-block copolymer in which a polyacrylonitrile or a poly(meth)acrylate block is prepared first, then a styrene or butadiene block is attached thereto, and so on.

Furthermore, there is no linking group required for joining the different blocks of the present block copolymer. One can simply add successive monomers to form successive blocks.

A plurality of specifically designed polymers and copolymers are accessible by the present invention, such as star and graft (co)polymers as described, inter alia, by C. J. Hawker in Angew. Chemie, 1995, 107, pages 1623-1627, dendrimers as described by K. Matyaszewski et al. in Macrmolecules 1996, Vol 29, No. 12, pages 4167-4171, graft (co)polymers as described by C. J. Hawker et al. in Macromol. Chem. Phys. 198, 155-166(1997), random copolymers as described by C. J. Hawker in Macromolecules 1996, 29, 2686-2688, or diblock and triblock copolymers as described by N. A. Listigovers in Macromolecules 196, 29, 8992-8993.

Yet another object of the present invention is a polymer or oligomer, containing at least one initiator group -CR₁R₂R₃ and at least one oxyamine group of formula (IIa) 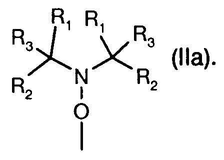 R₁, R₂ and R₃ have the meanings and preferred meanings as defined above, obtainable by the process as defined above.

In another of its aspects, this invention relates to a compound of formula (I) 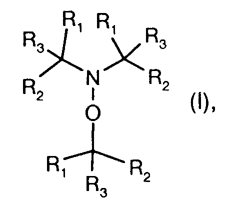
wherein R₁, R₂, R₃ are each independently of one another hydrogen, halogen, NO₂, cyano, -CONR₅R₆, -(R₉)COOR₄, -C(O)-R₇, -OR₈, -SR₈, -NHR₈, -N(R₈)₂, carbamoyl, di(C₁-C₁₈alkyl)carbamoyl, -C(=NR₅)(NHR₆);
unsubstituted C₁-C₁₈alkyl, C₂-C₁₈alkenyl, C₂-C₁₈alkynyl, C₇-C₉phenylalkyl, C₃-C₁₂cycloalkyl or C₂-C₁₂heterocycloalkyl; or
C₁-C₁₈alkyl, C₂-C₁₈alkenyl, C₂-C₁₈ alkynyl, C₇-C₉phenylalkyl, C₃-C₁₂cycloalkyl or C₂₋ C₁₂heterocycloalkyl, which are substituted by NO₂, halogen, amino, hydroxy, cyano, carboxy, C₁-C₄alkoxy, C₁-C₄alkylthio, C₁-C₄alkylamino or di(C₁-C₄alkyl)amino; or
phenyl, naphthyl, phenanthryl, anthryl, 5,6,7,8-tetrahydro-2-naphthyl, pyrrolyl, imidazolyl, pyrazolyl, pyrazinyl, pyrimidinyl, pyridazinyl, indolizinyl, isoindolyl, indolyl, indazolyl, purinyl, quinolizinyl, isoquinolyl, quinolyl, quinoxalinyl, quinazolinyl, cinnolinyl, pteridinyl, phenazinyl, isothiazolyl, phenothiazinyl, isoxazolyl, furazanyl, biphenyle or phenoxazinyl, which are unsubstituted or substituted by C₁-C₄alkyl, C₁-C₄alkoxy, C₁-C₄alkylthio, halogen, cyano, hydroxy, carboxy, C₁-C₄alkylamino or di(C₁-C₄alkyl)amino;
or R₂ and R₃, together with the linking carbon atom, form a C₃-C₁₂ cycloalkyl radical, a (C₄-C₁₂cycloalkanon)-yl radical or a C₂-C₁₂heterocycloalkyl radical with at least one O atom and/or a NR₈ group;
R₄ is hydrogen, C₁-C₁₈alkyl, phenyl, an alkali metal cation or a tetraalkylammonium cation;
R₅ and R₆ are hydrogen, C₁-C₁₈alkyl, C₂-C₁₈alkyl which is substituted by at least one hydroxy group or, taken together, form a C₂-C₁₂alkylene bridge or a C₂-C₁₂-alkylene bridge interrupted by at least one O or/and NR₈ atom;
R₇ is hydrogen, C₁-C₁₈alkyl or phenyl;
R₈ is C₁-C₁₈alkyl or C₂-C₁₈alkyl which is substituted by at least one hydroxy group; R₉ is C₁-C₁₂alkylen or a direct bond;
or R₁, R₂ and R₃ form together the residue of a polycyclic cycloaliphatic ring system or a polycyclic heterocycloaliphatic ring system with at least one di- or trivalent nitrogen atom;
with the proviso that,
if R₂ and R₃ are methyl, R₁ is not methyl, hydroxymethyl, -C(O)O-CH₃, -C(O)O-C₂H₅, NO₂ or CN;
if R₂ and R₃, together with the linking carbon atom, form a C₅- or C₆cycloalkyl radical, R₁ is not -CN or -C(O)OCH₃,
if R₂ and R₃ together are cyclopentanone-yl, R₁ is not methyl and
R₁, R₂ and R₃ are not F at the same time.

Preferred compounds are those wherein R₁, R₂, R₃ are each independently of one another NO₂, cyano, -(R₉)COOR₄, -CONR₅R₆, -C(O)-R₇, -OR₈, carbamoyl, di(C₁-C₈alkyl)carbamoyl, - C(=NR₅)(NHR₆);
unsubstituted C₁-C₈alkyl, C₇-C₉phenylalkyl, C₅-C₇cycloalkyl or C₄-C₇heterocycloalkyl; or
C₁-C₈alkyl, C₇-C₉phenylalkyl, C₅-C₇cycloalkyl or C₄-C₇heterocycloalkyl, which are substituted by amino, hydroxy, cyano, carboxy, C₁-C₄alkoxy, C₁-C₄alkylamino or di(C₁-C₄alkyl)amino;
or phenyl, naphthyl, pyrrolyl, imidazolyl, pyrazolyl, pyrazinyl, pyrimidinyl, pyridazinyl, which are unsubstituted or substituted by C₁-C₄alkyl, C₁-C₄alkoxy, C₁-C₄alkylthio, halogen, cyano, - hydroxy, carboxy, C₁-C₄alkylamino or di(C₁-C₄alkyl)amino;
or R₂ and R₃, together with the linking carbon atom, form a C₅-C₇cycloalkyl radical;
R₄ is C₁-C₈alkyl, phenyl, an alkali metal cation or a tetraalkylammonium cation;
R₅ and R₆ are hydrogen, C₁-C₈alkyl, C₂-C₈alkyl which is substituted by at least one hydroxy group or, taken together, form a C₁-C₆alkylene bridge;
R₇ is hydrogen, C₁-C₈alkyl or phenyl;
R₈ is C₁-C₈alkyl or C₂-C₈alkyl which is substituted by at least one hydroxy group;
R₉ is C₁-C₄alkylen or a direct bond;
or R₁, R₂ and R₃ form together the residue of a polycyclic cycloaliphatic ring system or a polycyclic heterocycloaliphatic ring system with at least one di- or trivalent nitrogen atom;
with the proviso that,
if R₂ and R₃ are methyl, R₁ is not methyl, hydroxymethyl, -C(O)O-CH₃, -C(O)C-C₂H₅, NO₂ or CN;
if R₂ and R₃, together with the linking carbon atom, form a C₅- or C₆cycloalkyl radical, R₁ is not -CN or -C(O)OCH₃,
if R₂ and R₃ together are cyclopentanone-yl, R₁ is not methyl and
R₁, R₂ and R₃ are not F at the same time.

Particularly preferred is a compound wherein R₁ is NO₂, cyano, -CONR₅R₆, - (R₉)COOR₄, - C(=NR₅)(NHR₆);
R₂ and R₃ are each independently of one another unsubstituted C₁-C₈alkyl, C₅-C₇cycloalkyl or phenyl;
or R₂ and R₃, together with the linking carbon atom, form a C₅-C₇ cycloalkyl radical;
R₄ is hydrogen, C₁-C₈alkyl, phenyl;
R₅ and R₆ are hydrogen, C₁-C₈alkyl, C₂-C₈alkyl which is substituted by at least one hydroxy group or, taken together, form a C₂-C₆alkylene bridge;
R₉ is C₁-C₄alkylen or a direct bond;
with the proviso that,
if R₂ and R₃ are methyl, R₁ is not methyl, hydroxymethyl, -C(O)O-CH₃, -C(O)O-C₂H₅, NO₂ or CN;
if R₂ and R₃, together with the linking carbon atom, form a C₅- or C₆cycloalkyl radical, R₁ is not -CN or -C(O)OCH₃,
if R₂ and R₃ together are cyclopentanone-yl, R₁ is not methyl and
R₁, R₂ and R₃ are not F at the same time.

Further meanings and preferrences for the different substitutents R₁ to R₉ are mentioned before.

The invention is also directed to a compound of formula (IV) which is an intermediate in the process for preparing (co)polymers and in the manufacture of a compound of formula (I) 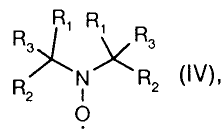 wherein R₁, R₂, R₃ are each independently of one another hydrogen, halogen, NO₂, cyano,
-CONR₅R₆, -(R₉)COOR₄, -C(O)-R₇, -OR₈, -SR₈, -NHR₈, -N(R₈)₂, carbamoyl, di(C₁-C₁₈alkyl)carbamoyl, -C(=NR₅)(NHR₆);
unsubstituted C₁-C₁₈alkyl, C₂-C₁₈alkenyl, C₂-C₁₈alkynyl, C₇-C₉phenylalkyl, C₃-C₁₂cycloalkyl or C₂-C₁₂heterocycloalkyl; or
C₁-C₁₈alkyl, C₂-C₁₈alkenyl, C₂-C₁₈ alkynyl, C₇-C₉phenylalkyl, C₃-C₁₂cycloalkyl or C₂-C₁₂heterocycloalkyl, which are substituted by NO₂, halogen, amino, hydroxy, cyano, carboxy, C₁-C₄alkoxy, C₁-C₄alkylthio, C₁-C₄alkylamino or di(C₁-C₄alkyl)amino; or
phenyl, naphthyl, phenanthryl, anthryl, 5,6,7,8-tetrahydro-2-naphthyl, pyrrolyl, imidazolyl, pyrazolyl, pyrazinyl, pyrimidinyl, pyridazinyl, indolizinyl, isoindolyl, indolyl, indazolyl, purinyl, quinolizinyl, isoquinolyl, quinolyl, quinoxalinyl, quinazolinyl, cinnolinyl, pteridinyl, phenazinyl, isothiazolyl, phenothiazinyl, isoxazolyl, furazanyl, biphenyle or phenoxazinyl, which are unsubstituted or substituted by C₁-C₄alkyl, C₁-C₄alkoxy, C₁-C₄alkylthio, halogen, cyano, hydroxy, carboxy, C₁-C₄alkylamino or di(C₁-C₄alkyl)amino;
or R₂ and R₃, together with the linking carbon atom, form a C₃-C₁₂cycloalkyl radical, a (C₄-C₁₂cycloalkanon)-yl radical or a C₂-C₁₂heterocycloalkyl radical with at least one O atom and/or a NR₈ group;
R₄ is C₁-C₁₈alkyl, phenyl, an alkali metal cation or a tetraalkylammonium cation;
R₅ and R₆ are hydrogen, C₁-C₁₈alkyl, C₂-C₁₈alkyl which is substituted by at least one hydroxy group or, taken together, form a C₂-C₁₂alkylene bridge or a C₂-C₁₂-alkylene bridge interrupted by at least one O or/and NR₈ atom;
R₇ is hydrogen, C₁-C₁₈alkyl or phenyl;
R₈ is C₁-C₁₈alkyl or C₂-C₁₈alkyl which is substituted by at least one hydroxy group;
R₉ is C₁-C₄alkylen or a direct bond;
or R₁, R₂ and R₃ form together the residue of a polycyclic cycloaliphatic ring system or a polycyclic heterocycloaliphatic ring system with at least one di- or trivalent nitrogen atom and
with the proviso that,
if R₂ and R₃ are methyl, R₁ is not methyl, hydroxymethyl, -C(O)O-CH₃, -C(O)O-C₂H₅, NO₂ or CN;
if R₂ and R₃, together with the linking carbon atom, form a C₅ or C₆cycloalkyl radical, R₁ is not -CN or -C(O)OCH₃
if R₂ and R₃ together are cyclopentanone, R, is not methyl and
R₁, R₂ and R₃ are not F at the same time.

Meanings and preferrences for the different substitutents R₁ to R₉ are already mentioned.

A further object of the present invention is a process for preparing a compound of formula (I) 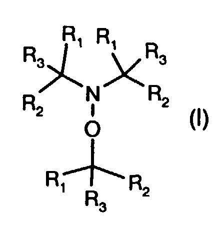 by generating a free radical ·CR₁R₂R₃ from a compound capable of eliminating a neutral molecule, or undergoing C-C bond-scission upon thermal or photochemical treatment, or by hydrogen abstraction from a compound R₁R₂R₃C-H in reaction with reactive radicals, and reacting the free radical ·CR₁R₂R₃ with NO in a solvent which does not interfere with the radical reaction.

Examples for neutral molecules which can be eliminated are N₂ or O₂. Reactive radicals which are able to abstract hydrogen are for example alkoxy radicals.

Suitable solvents are aromatic, aliphatic or cycloaliphatic hydrocarbons, such as toluene, benzene xylene, octane or cyclohexane, ethers, such as dioxane, tetrahydrofurane or dibutylether, alcohols, glycols or esters and amides of carboxylic acids.

Preferred is a process the free radical ·CR₁R₂R₃ is prepared by heating or irradiating a compound of formula IIIa, IIIb or IIIc

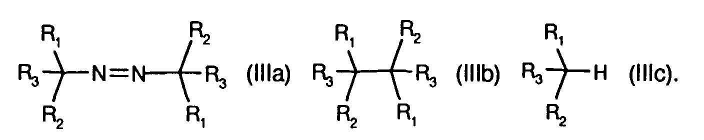

Preferably the radical ·CR₁R₂R₃ is prepared by heating one of these compounds from 20° to 150° C, more preferrably from 50° to 150° C and most preferrably from 70° to 140° C. R₁, R₂ and R₃ have the meanings and preferred meanings defined above.

The reaction of reacting free radicals with NO is known per se and described by B. A. Gingras et al. in J. Chem. Soc. page 1920, 1954.

The production of C-centered radicals is described, inter alia, in Houben Weyl, Methoden der Organischen Chemie, Vol. E 19a, pages 60-147. These methods can be applied in general analogy.

Most preferably, the free radical source is 2,2'-azobisisobutyronitrile, 2,2'-azobis(2-methylbutyronitrile), 2,2'-azobis(2,4-dimethylvaleronitrile), 2,2'-azobis(4-methoxy-2,4-dimethylvaleronitrile), 1,1'-azobis(1-cyclohexanecarbonitrile), 2,2'-azobis(isobutyramide) dihydrate, 2-phenylazo-2,4-dimethyl-4-methoxyvaleronitrile, dimethyl-2,2'-azobisisobutyrate, 2-(carbamoylazo)isobutyronitrile, 2,2'-azobis(2,4,4-trimethylpentane), 2,2'-azobis(2-methylpropane), 2,2'-azobis(N,N'-dimethyleneisobutyramidine), free base or hydrochloride, 2,2'-azobis(2-amidinopropane), free base or hydrochloride, 2,2'-azobis{2-methyl-N-[1,1-bis(hydroxymethyl)ethyl]propionamide} or 2,2'-azobis{2-methyl-N-[1,1-bis(hydroxymethyl)-2-hydroxyethyl]propionamide.

These compounds are commercially available.

If more than one radical source is used, a mixture of substitution patterns is obtainable.

This invention also relates to the use of an initiator compound of formula (I) for polymerizing ethylenically unsaturated monomers.

The alkoxyamines of formula (I) may be prepared and isolated as described above. However, it is also possible with the disadvantages described before to produce the compounds of formula (I) in situ during polymerization by adding a compound of formula IV, which has been isolated as intermediate in the process described before, to the polymerizable monomers and by adding, also in situ, the corresponding radical initiator.

Consequently a further aspect of the invention is a polymerizable composition, comprising
a) at least one ethylenically unsaturated monomer or oligomer,
b) a compound of formula (IV) and c) a radical iniator as described above capable of generating a free radical of formula (V) 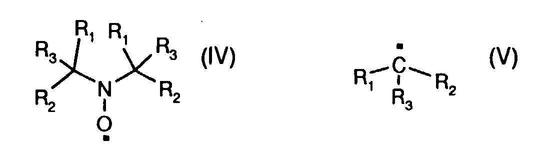
wherein R₁, R₂, R₃ are each independently of one another hydrogen, halogen, NO₂, cyano, -CONR₅R₆, -(R₉)COOR4, -C(O)-R₇, -OR₈, -SR₈, -NHR₈, -N(R₈)₂, carbamoyl, di(C₁-C₁₈alkyl)carbamoyl, -C(=NR₅)(NHR₆);
unsubstituted C₁-C₁₈alkyl, C₂-C₁₈alkenyl, C₂-C₁₈alkynyl, C₇-C₉phenylalkyl, C₃-C₁₂cycloalkyl or C₂-C₁₂heterocycloalkyl; or
C₁-C₁₈alkyl, C₂-C₁₈alkenyl, C₂-C₁₈ alkynyl, C₇-C₉phenylalkyl, C₃-C₁₂cycloalkyl or C₂-C₁₂heterocycloalkyl, which are substituted by NO₂, halogen, amino, hydroxy, cyano, carboxy, C₁-C₄alkoxy, C₁-C₄alkylthio, C₁-C₄alkylamino or di(C₁-C₄alkyl)amino; or
phenyl, naphthyl, phenanthryl, anthryl, pyrrolyl, imidazolyl, pyrazolyl, pyrazinyl, pyrimidinyl, pyridazinyl, indolizinyl, isoindolyl, indolyl, indazolyl, purinyl, quinolizinyl, isoquinolyl, quinolyl, quinoxalinyl, quinazolinyl, cinnolinyl, pteridinyl, phenazinyl, isothiazolyl, phenothiazinyl, isoxazolyl, furazanyl, biphenyle or phenoxazinyl, which are unsubstituted or substituted by C₁-C₄alkyl, C₁-C₄alkoxy, C₁-C₄alkylthio, halogen, cyano, hydroxy, carboxy, C₁-C₄alkylamino or di(C₁-C₄alkyl)amino;
or R₂ and R₃, together with the linking carbon atom, form a C₃-C₁₂cycloalkyl radical, a (C₄-C₁₂cycloalkanon)-yl radical or a C₂-C₁₂heterocycloalkyl radical containing at least one O atom and/or a NR₈ group;
R₄ is hydrogen, C₁-C₁₈alkyl, phenyl, an alkali metal cation or a tetraalkylammonium cation;
R₅ and R₆ are hydrogen, C₁-C₁₈alkyl, C₂-C₁₈alkyl which is substituted by at least one hydroxy group or, taken together, form a C₂-C₁₂alkylene bridge or a C₂-C₁₂-alkylene bridge interrupted by at least one O or/and NR₈ atom;
R₇ is hydrogen, C₁-C₁₈alkyl or phenyl;
R₈ is hydrogen, C₁-C₁₈alkyl or C₂-C₁₈alkyl which is substituted by at least one hydroxy group;
R₉ is C₁-C₁₂alkylen;
or R₁, R₂ and R₃ form together the residue of a polycyclic cycloaliphatic ring system or a polycyclic heterocycloaliphatic ring system with at least one di- or trivalent nitrogen atom.

Still another aspect of the invention is a process for preparing an oligomer, a cooligomer, a polymer or a copolymer (block or random) by free radical polymerization of at least one ethylenically unsaturated monomer/oligomer, which comprises subjecting the above composition to heat or actinic radiation.

Suitable initiators are those listed above including their preferrences.

The following Examples illustrate the invention in more detail.

### A) Examples for the preparation of N,N,O-trisubstituted hydroxylamines from NO

According to B. A. Gingras, W. A. Waters: J. Chem. Soc. 1920 (1954), the compounds presented in Table 1 have been prepared.

### Preparation of compound (101)

15.6 g (0.064 Mol) of 1,1'-Azobis-(cyclohexanecarbonitrile) are dissolved in 150 ml of toluene. After careful purge with nitrogen, a slow stream of nitrogen monoxide is bubbled continuously during 5¹/₂ h through the stirred solution held at 110 °C. About 100 ml of toluene are then distilled off on the rotary evaporator and the precipitate of 1,1'-bis-(cyclohexanecarbonitrile) is filtered off. The filtrate is evaporated and subjected to column chromatography on silica gel (methylene chloride - hexane 1:1 to 2:1). The pure fractions are recrystallized from methanol to give 5.4 g of (101).

### Preparation of compound (102)

20 g (0.122 Mo) of 1,1'-Azobis-isobutyronitrile are dissolved in 150 ml of toluene. After careful purge with nitrogen, a slow stream of nitrogen monoxide is bubbled continuously during 3¹/₂ h through the stirred solution held at 90 °C. Toluene is then distilled off on the rotary evaporator and the residue is subjected to column chromatography on silica gel (methylene chloride - hexane 3:2). The pure fractions are recrystallized from methyl-tert.-butylether to give 5.6 g of (102).

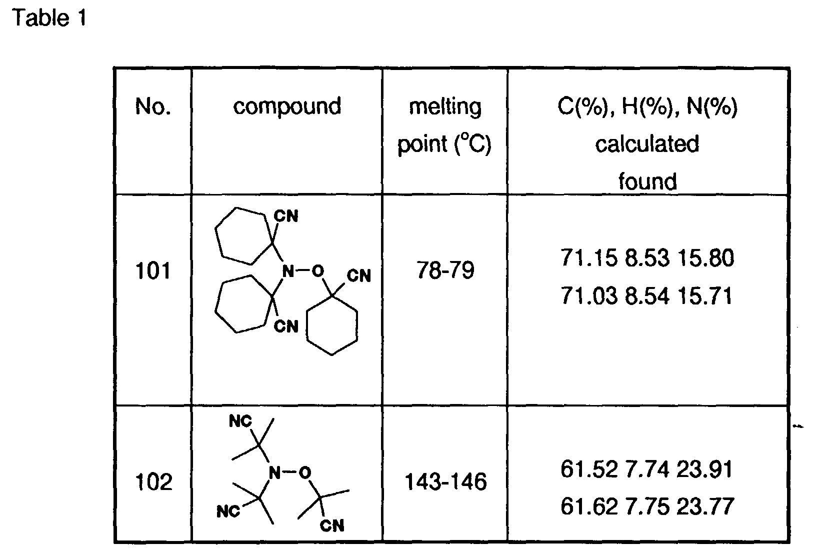

### B) Polymerizations using compounds of Table I as initiators

### General remarks:

Solvents and monomers are distilled over a Vigreux column under argon atmosphere or under vacuum, shortly before being used.

To remove oxygen all polymerization reaction mixtures are flushed before polymerization with argon and evacuated under vaccum applying a freeze-thaw cycle. The reaction mixtures are then polymerized under argon atmosphere.

At the start of the polymerization reaction, all starting materials are homogeneously dissolved.

Conversion is determined by removing unreacted monomers from the polymer at 80° C and 0.002 torr for 30 minutes , weighing the remaining polymer and subtract the weight of the initiator.

Characterization of the polymers is carried out by MALDI-MS (Matrix Assisted Laser Desorption Ionization Mass Spectrometry) and/or GPC (Gel Permeation Chromatography).
MALDI-MS: Measurements are performed on a linear TOF (Time Of Flight) MALDI-MS LDI-1700 Linear Scientific Inc. , Reno, USA. The matrix is 2,5-dihydroxybenzoic acid and the laser wavelength is 337 nm.
GPC: Is performed using RHEOS 4000 of FLUX INSTRUMENTS. Tetrahydrofurane (THF) is used as a solvent and is pumped at 1 ml/min. Two chromatography columns are put in series: type Plgel 5µm mixed-C of POLYMER INSTRUMENTS, Shropshire, UK. Measurements are performed at 40 °C. The columns are calibrated with low polydispersity polystyrenes having Mn from 200 to 2 000 000 Dalton. Detection is carried out using a RI-Detector ERC-7515A of ERCATECH AG at 30 °C.

### Polymerization of n-butylacrylate using compound 102

In a 50 ml three neck flask, equipped with thermometer, cooler and magnetic stirrer, 548 mg (2,34 mmol) of compound 102 and 20 g (156 mmol) of n-butylacrylate are mixed and degased. The clear solution obtained is heated under argon to 115 °C. Polymerization starts spontaneously and the temperature rises to 140° C. After 15 minutes, the exothermal reaction slowly stops and the viscosity of the solution increases. The reaction mixture is stirred for an additional 20 minutes at 145 °C and is then cooled to 80 °C. The remaining monomer is removed by evaporation under high vacuum. 19,2 g (96%) of the initial monomer have reacted. A clear colourless viscous fluid is obtained.
GPC: Mn = 6700, Mw = 17000, polydispersity = 2,5.

## Claims

1. A polymerizable composition, comprising
a) at least one ethylenically unsaturated monomer or oligomer, and
b) an initiator compound of formula (I) 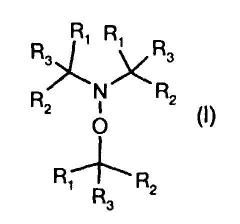
wherein R₁, R₂, R₃ are each independently of one another hydrogen, halogen, NO₂, cyano, -CONR₅R₆, -(R₉)COOR4, -C(O)R₇, -OR₈, -SR₈, -NHR₈, -N(R₈)₂, carbamoyl, di(C₁-C₁₈alkyl)carbamoyl, -C(=NR₅)(NHR₆);
unsubstituted C₁-C₁₈alkyl, C₂-C₁₈alkenyl, C₂-C₁₈alkynyl, C₇-C₉phenylalkyl, C₃-C₁₂cycloalkyl or C₂-C₁₂heterocycloalkyl; or
C₁-C₁₈alkyl, C₂-C₁₈alkenyl, C₂-C₁₈ alkynyl, C₇-C₉phenylalkyl, C₃-C₁₂cycloalkyl or C₂-C₁₂heterocycloalkyl, which are substituted by NO₂, halogen, amino, hydroxy, cyano, carboxy, C₁₋C₄alkoxy, C₁₋C₄alkylthio, C₁₋C₄alkylamino or di(C₁₋C₄alkyl)amino; or
phenyl, naphthyl, phenanthryl, anthryl, pyrrolyl, imidazolyl, pyrazolyl, pyrazinyl, pyrimidinyl, pyridazinyl, indolizinyl, isoindolyl, indolyl, indazolyl, purinyl, quinolizinyl, isoquinolyl, quinolyl, quinoxalinyl, quinazolinyl, cinnolinyl, pteridinyl, phenazinyl, isothiazolyl, phenothiazinyl, isoxazolyl, furazanyl, biphenyle or phenoxazinyl, which are unsubstituted or substituted by C₁₋C₄alkyl, C₁₋C₄alkoxy, C₁₋C₄alkylthio, halogen, cyano, hydroxy, carboxy, C₁₋C₄alkylamino or di(C₁₋C₄alkyl)amino;
or R₂ and R₃, together with the linking carbon atom, form a C₃-C₁₂cycloalkyl radical, a (C₄-C₁₂cycloalkanon)-yl radical or a C₂₋C₁₂heterocycloalkyl radical containing at least one O atom and/or a NR₈ group;
R₄ is hydrogen, C₁-C₁₈alkyl, phenyl, an alkali metal cation or a tetraalkylammonium cation;
R₅ and R₆ are hydrogen, C₁-C₁₈alkyl, C₂-C₁₈alkyl which is substituted by at least one hydroxy group or, taken together, form a C₂-C₁₂alkylene bridge or a C₂-C₁₂-alkylene bridge interrupted by at least one O or/and NR₈ atom;
R₇ is hydrogen, C₁-C₁₈alkyl or phenyl;
R₈ is hydrogen, C₁-C₁₈alkyl or C₂-C₁₈alkyl which is substituted by at least one hydroxy group;
R₉ is C₁-C₁₂alkylen or a direct bond;
or R₁, R₂ and R₃ form together the residue of a polycyclic cycloaliphatic ring system or a polycyclic heterocycloaliphatic ring system with at least one di-or trivalent nitrogen atom.

2. A composition according to claim 1, which additionally comprises a solvent selected from the group consisting of water, alcohols, esters, ethers, ketones, amides, sulfoxides, hydrocarbons and halogenated hydrocarbons.

3. A composition according to claim 1, wherein the ethylenically unsaturated monomer or oligomer is selected from the group consisting of styrene, substituted styrene, conjugatecl dienes, acrolein, vinyl acetate, (alkyl)acrylic acidanhydrides, (alkyl)acrylic acid salts, (alkyl)acrylic esters or (alkyl)acrylamides.

4. A composition according to claim 3 wherein the ethylenically unsaturated monomer is styrene, α-methyl styrene, p-methyl styrene or a compound of formula CH₂=C(Rₐ)-(C=Z)-R_{b}, wherein Rₐ is hydrogen or C₁-C₄alkyl, R_{b} is, NH₂, O(Me), glycidyl, unsubstituted C₁-C₁₈alkoxy or hydroxy-substituted C₁-C₁₈alkoxy, unsubstituted C₁-C₁₈alkylamino, di(C₁-C₁₈alkyl)amino, hydroxy-substituted C₁-C₁₈alkylamino or hydroxy-substituted di(C₁-C₁₈alkyl)amino;
Me is a metal atom, t is 1 or 2, and
Z is oxygen or sulfur.

5. A composition according to claim 4, wherein Rₐ is hydrogen or methyl, R_{b} is NH₂, gycidyl, unsubstituted or with hydroxy substituted C₁-C₄alkoxy, unsubstituted C₁-C₄alkylamino, di(C₁-C₄alkyl)amino, hydroxy-substituted C₁-C₄alkylamino or hydroxy-substituted di(C₁-C₄alkyl)amino;and
Z is oxygen.

6. A polymerizable composition according to claim 5, wherein the ethylenically unsaturated monomer is methylacrylate, ethylacrylate, butylacrylate, isobutylacrylate, tert. butylacrylate, hydroxyethylacrylate, hydroxypropylacrylate, dimethylaminoethylacrylate, glycidylacrylates, methyl(meth)acrylate, ethyl(meth)acrylate, butyl(meth)acrylate, hydroxyethyl(meth)acrylate, hydroxypropyl(meth)acrylate, dimethylaminoethyl(meth)acrylate, glycidyl(meth)acrylates, acrylonitrile, acrylamide or methacrylamide.

7. A composition according to claim 1, wherein
R₁, R₂, R₃ are each independently of one another NO₂, cyano, -(R₉)COOR₄, -CONR₅R₆, - C(O)-R₇, -OR₈, carbamoyl, di(C₁-C₁₈alkyl)carbamoyl, -C(=NR₅)(NHR₆);
unsubstituted C₁-C₁₈alkyl, C₇-C₉phenylalkyl, C₃-C₁₂cycloalkyl or C₂-C₁₂heterocycloalkyl; or C₁-C₁₈alkyl, C₇-C₉phenylalkyl, C₃-C₁₂cycloalkyl or C₂-C₁₂heterocycloalkyl, which are substituted by amino, hydroxy, cyano, carboxy, C₁-C₄alkoxy, C₁-C₄alkylamino or di(C₁-C₄alkyl)amino; or
phenyl, naphthyl, phenanthryl, anthryl, pyrrolyl, imidazolyl, pyrazolyl, pyrazinyl, pyrimidinyl, pyridazinyl, indolizinyl, isoindolyl, indolyl, indazolyl, purinyl, quinolizinyl, isoquinolyl, quinolyl, quinoxalinyl, quinazolinyl, cinnolinyl, pteridinyl, phenazinyl, isothiazolyl, phenothiazinyl, isoxazolyl, furazanyl, biphenyle or phenoxazinyl, which are unsubstituted or substituted by C₁-C₄alkyl, C₁-C₄alkoxy, C₁-C₄alkylthio, halogen, cyano, hydroxy, carboxy, C₁-C₄alkylamino or di(C₁-C₄alkyl)amino;
or R₂ and R₃, together with the linking carbon atom, form a C₃-C₁₂cycloalkyl radical;
R₄ is, C₁-C₁₈alkyl, phenyl, an alkali metal cation or a tetraalkylammonium cation;
R₅ and R₆ are hydrogen, C₁-C₁₈alkyl, C₂-C₁₈alkyl which is substituted by at least one hydroxy group or, taken together, form a C₂-C₁₂alkylene bridge;
R₇ is hydrogen, C₁-C₁₈alkyl or phenyl;
R₈ is C₁-C₁₈alkyl or C₂-C₁₈alkyl which is substituted by at least one hydroxy group;
R₉ is C₁-C₄alkylen or a direct bond;
or R₁, R₂ and R₃ form together the residue of a polycyclic cycloaliphatic ring system or a polycyclic heterocycloaliphatic ring system with at least one di- or trivalent nitrogen atom.

8. A composition according to claim 7, wherein
R₁, R₂, R₃ are each independently of one another NO₂, cyano, -(R₉)COOR₄, -CONR₅R₆, - C(O)-R₇, -OR₈, carbamoyl, di(C₁-C₈alkyl)carbamoyl, -C(=NR₅)(NHR₆);
unsubstituted C₁-C₈alkyl, C₇-C₉phenylalkyl, C₅-C₇cycloalkyl or C₄-C₇heterocycloalkyl; or
C₁-C₈alkyl, C₇-C₉phenylalkyl, C₅-C₇cycloalkyl or C₄-C₇heterocycloalkyl, which are substituted by amino, hydroxy, cyano, carboxy, C₁-C₄alkoxy, C₁-C₄alkylamino or di(C₁-C₄alkyl)amino;
or phenyl, naphthyl, pyrrolyl, imidazolyl, pyrazolyl, pyrazinyl, pyrimidinyl, pyridazinyl, which are unsubstituted or substituted by C₁-C₄alkyl, C₁-C₄alkoxy, C₁-C₄alkylthio, halogen, cyano, hydroxy, carboxy, C₁-C₄alkylamino or di(C₁-C₄alkyl)amino;
or R₂ and R₃, together with the linking carbon atom, form a C₅-C₇cycloalkyl radical;
R₄ is C₁-C₈alkyl, phenyl, an alkali metal cation or a tetraalkylammonium cation;
R₅ and R₆ are hydrogen, C₁-C₈alkyl, C₂-C₈alkyl which is substituted by at least one hydroxy group or, taken together, form a C₂-C₆alkylene bridge;
R₇ is hydrogen, C₁-C₈alkyl or phenyl;
R₈ is C₁-C₈alkyl or C₂-C₈alkyl which is substituted by at least one hydroxy group;
R₉ is C₁-C₄alkylen or a direct bond;
or R₁, R₂ and R₃ form together the residue of a polycyclic cycloaliphatic ring system or a polycyclic heterocycloaliphatic ring system with at least one di-or trivalent nitrogen atom.

9. A composition according to claim 8, wherein
R₁, R₂, R₃ are each independently of one another NO₂, cyano, -(R₉)COOR₄, - CONR₅R₆, C(O)-R₇, -OR₈, carbamoyl, di(C₁-C₁₈alkyl)carbamoyl, -C(=NR₅)(NHR₆);
unsubstituted C₁-C₈alkyl or C₅-C₇cycloalkyl;
or phenyl, which is unsubstituted or substituted by C₁-C₄alkyl, C₁-C₄alkoxy, cyano, hydroxy, carboxy, C₁-C₄alkylamino or di(C₁-C₄alkyl)amino;
or R₂ and R₃, together with the linking carbon atom, form a C₅-C₇cycloalkyl radical;
R is C₁-C₈alkyl, phenyl, an alkali metal cation or a tetraalkylammonium cation;
R₅ and R₆ are hydrogen, C₁-C₈alkyl, C₂-C₈alkyl which is substituted by at least one hydroxy group or, taken together, form a C₂-C₆alkylene bridge;
R₇ is, C₁-C₈alkyl or phenyl;
R₈ is C₁-C₈alkyl or C₂-C₈alkyl which is substituted by at least one hydroxy group;
R₉ is C₁-C₄alkylen or a direct bond.

10. A composition according to claim 9, wherein
R₁ is NO₂, cyano, -CONR₅R₆, -(R₉)COOR₄, -C(=NR₅)(NHR₆);
R₂ and R₃ are each independently of one another unsubstituted C₁-C₈alkyl, C₅-C₇cycloalkyl or phenyl;
or R₂ and R₃, together with the linking carbon atom, form a C₅-C₇cycloalkyl radical;
R₄ is, C₁-C₈alkyl, phenyl;
R₅ and R₆ are hydrogen, C₁-C₈alkyl, C₂-C₈alkyl which is substituted by at least one hydroxy group or, taken together, form a C₂-C₆alkylene bridge;
R₉ is C₁-C₄alkylen or a direct bond.

11. A composition according to claim 1 wherein the group

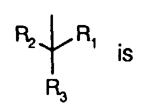

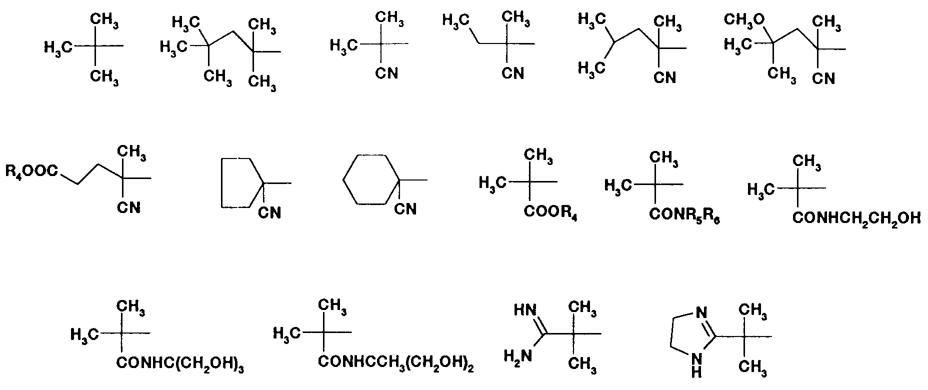

12. A process for preparing an oligomer, a cooligomer, a polymer or a copolymer (block or random) by free radical polymerization of at least one ethylenically unsaturated monomer/oligomer, which comprises (co)polymerizing the monomer or monomers/oligomers in the presence of an initiator compound of formula (I) according to claim 1 under reaction conditions capable of effecting scission of the O-C bond to form two free radicals, the radical ·CR₁R₂R₃ being capable of initiating polymerization.

13. A process according to claim 12, wherein the scission of the O-C bond is effected by ultrasonic treatment, heating or exposure to electromagnetic radiation, ranging from γ to microwaves.

14. A process according to claim 12, wherein the scission of the O-C bond is effected by heating and takes place at a temperature of between 25°C and 150°C.

15. A process according to claim 12 for preparing a block copolymer involving at least two stages, which comprises forming a polymer with alkoxyamine end groups of the general structure of formula II 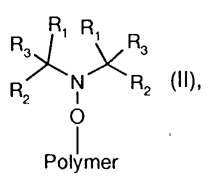 wherein R₁, R₂ and R₃ are as defined in claim 1, the polymer containing the initiator group -CR₁R₂R₃ and having the oxyamine group essentially attached as terminal group, and adding a further monomer followed by heating to form a block copolymer.

16. A polymer or oligomer, containing at least one initiator group -CR₁R₂R₃ and at least one oxyamine group of formula (IIa) 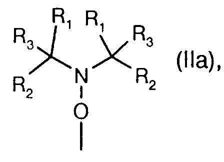 wherein R₁, R₂ and R₃ are as defined in claim 1, obtainable by the process according to claim 12.

17. A compound of formula (I) 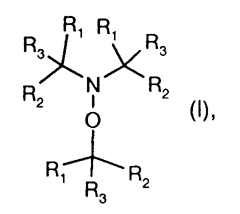
wherein R₁, R₂, R₃ are each independently of one another hydrogen, halogen, NO₂, cyano, -CONR₅R₆, -(R₉)COOR₄, -C(O)-R₇, -OR₈, -SR₈, -NHR₈, -N(R₈)₂, carbamoyl, di(C₁-C₁₈alkyl)carbamoyl, -C(=NR₅)(NHR₆);
unsubstituted C₁-C₁₈alkyl, C₂-C₁₈alkenyl, C₂-C₁₈alkynyl, C₇-C₉phenylalkyl, C₃-C₁₂cycloalkyl or C₂-C₁₂heterocycloalkyl; or
C₁-C₁₈alkyl, C₂-C₁₈alkenyl, C₂-C₁₈ alkynyl, C₇-C₉phenylalkyl, C₃-C₁₂cycloalkyl or C₂-C₁₂heterocycloalkyl, which are substituted by NO₂, halogen, amino, hydroxy, cyano, carboxy, C₁-C₄alkoxy, C₁-C₄alkylthio, C₁-C₄alkylamino or di(C₁-C₄alkyl)amino; or
phenyl, naphthyl, phenanthryl, anthryl, 5,6,7,8-tetrahydro-2-naphthyl, pyrrolyl, imidazolyl, pyrazolyl, pyrazinyl, pyrimidinyl, pyridazinyl, indolizinyl, isoindolyl, indolyl, indazolyl, purinyl, quinolizinyl, isoquinolyl, quinolyl, quinoxalinyl, quinazolinyl, cinnolinyl, pteridinyl, phenazinyl, isothiazolyl, phenothiazinyl, isoxazolyl, furazanyl, biphenyle or phenoxazinyl, which are unsubstituted or substituted by C₁-C₄alkyl, C₁-C₄alkoxy, C₁-C₄alkylthio, halogen, cyano, hydroxy, carboxy, C₁-C₄alkylamino or di(C₁-C₄alkyl)amino;
or R₂ and R₃, together with the linking carbon atom, form a C₃-C₁₂cycloalkyl radical, a (C₄-C₁₂cycloalkanon)-yl radical or a C₂-C₁₂heterocycloalkyl radical with at least one O atom and/or a NR₈ group;
R₄ is hydrogen, C₁-C₁₈alkyl, phenyl, an alkali metal cation or a tetraalkylammonium cation;
R₅ and R₆ are hydrogen, C₁-C₁₈alkyl, C₂-C₁₈alkyl which is substituted by at least one hydroxy group or, taken together, form a C₂-C₁₂alkylene bridge or a C₂-C₁₂-alkylene bridge interrupted by at least one O or/and NR₈ atom;
R₇ is hydrogen, C₁-C₁₈alkyl or phenyl;
R₈ is C₁-C₁₈alkyl or C₂-C₁₈alkyl which is substituted by at least one hydroxy group;
R₉ is C₁-C₁₂alkylen or a direct bond;
or R₁, R₂ and R₃ form together the residue of a polycyclic cycloaliphatic ring system or a polycyclic heterocycloaliphatic ring system with at least one di-or trivalent nitrogen atom;
with the proviso that,
if R₂ and R₃ are methyl, R₁ is not methyl, hydroxymethyl, -C(O)O-CH₃, -C(O)O-C₂H₅, NO₂ or CN;
if R₂ and R₃, together with the linking carbon atom, form a C₅- or C₆cycloalkylradical, R₁ is not -CN or -C(O)OCH₃,
if R₂ and R₃ together are cyclopentanone-yl, R₁ is not methyl and
R₁, R₂ and R₃ are not F at the same time.

18. A compound according to claim 17 wherein
R₁, R₂, R₃ are each independently of one another NO₂, cyano, -(R₉)COOR₄, - CONR₅R₆, - C(O)-R₇, -OR₈, carbamoyl, di(C₁-C₈alkyl)carbamoyl, -C(=NR₅)(NHR₆);
unsubstituted C₁-C₈alkyl, C₇-C₉phenylalkyl, C₅-C₇cycloalkyl or C₄-C₇heterocycloalkyl; or
C₁-C₈alkyl, C₇-C₉phenylalkyl, C₅-C₇cycloalkyl or C₄-C₇heterocycloalkyl, which are substituted by amino, hydroxy, cyano, carboxy, C₁-C₄alkoxy, C₁-C₄alkylamino or di(C₁-C₄alkyl)amino;
or phenyl, naphthyl, pyrrolyl, imidazolyl, pyrazolyl, pyrazinyl, pyrimidinyl, pyridazinyl, which are unsubstituted or substituted by C₁-C₄alkyl, C₁-C₄alkoxy, C₁-C₄alkylthio, halogen, cyano, - hydroxy, carboxy, C₁-C₄alkylamino or di(C₁-C₄alkyl)amino;
or R₂ and R₃, together with the linking carbon atom, form a C₅-C₇ cycloalkyl radical;
R₄ is C₁-C₈alkyl, phenyl, an alkali metal cation or a tetraalkylammonium cation;
R₅ and R₆ are hydrogen, C₁-C₈alkyl, C₂-C₈alkyl which is substituted by at least one hydroxy group or, taken together, form a C₂-C₆alkylene bridge;
R₇ is hydrogen, C₁-C₈alkyl or phenyl;
R₈ is C₁-C₈alkyl or C₂-C₈alkyl which is substituted by at least one hydroxy group;
R₉ is C₁-C₄alkylen or a direct bond;
or R₁, R₂ and R₃ form together the residue of a polycyclic cycloaliphatic ring system or a polycyclic heterocycloaliphatic ring system with at least one di-or trivalent nitrogen atom;
with the proviso that,
if R₂ and R₃ are methyl, R₁ is not methyl, hydroxymethyl, -C(O)O-CH₃, -C(O)O-C₂H₅, NO₂ or CN;
if R₂ and R₃, together with the linking carbon atom, form a C₅- or C₆cycloalkyl radical, R₁ is not -CN or -C(O)OCH₃,
if R₂ and R₃ together are cyclopentanone-yl, R₁ is not methyl and
R₁, R₂ and R₃ are not F at the same time.

19. A compound according to claim 18 wherein
R₁ is NO₂, cyano, -CONR₅R₆, -(R₉)COOR₄, -C(=NR₅)(NHR₆);
R₂ and R₃ are each independently of one another unsubstituted C₁-C₈alkyl, C₅-C₇cycloalkyl or phenyl;
or R₂ and R₃, together with the linking carbon atom, form a C₅-C₇cycloalkyl radical;
R₄ is hydrogen, C₁-C₈alkyl, phenyl;
R₅ and R₆ are hydrogen, C₁-C₈alkyl, C₂-C₈alkyl which is substituted by at least one hydroxy group or, taken together, form a C₂-C₆alkylene bridge;
R₉ is C₁-C₄alkylen or a direct bond;
with the proviso that,
if R₂ and R₃ are methyl, R₁ is not methyl, hydroxymethyl, -C(O)O-CH₃, -C(O)O-C₂H₅, NO₂ or CN;
if R₂ and R₃, together with the linking carbon atom, form a C₅- or C₆cycloalkyl radical, R₁ is not -CN or -C(O)OCH₃,
if R₂ and R₃ together are cyclopentanone-yl, R₁ is not methyl and
R₁, R₂ and R₃ are not F at the same time.

20. A compound of formula (IV) 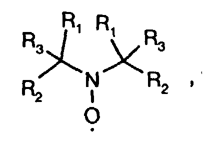 wherein
R₁, R₂, R₃ are each independently of one another hydrogen, halogen, NO₂, cyano, -CONR₅R₆ , -(R₉)COOR₄, -C(O)-R₇, -OR₈, -SR₈, -NHR₈, -N(R₈)₂, carbamoyl, di(C₁-C₁₈alkyl)carbamoyl, -C(=NR₅)(NHR₆);
unsubstituted C₁-C₁₈alkyl, C₂-C₁₈alkenyl, C₂-C₁₈alkynyl, C₇-C₉phenylalkyl, C₃-C₁₂cycloalkyl or C₂-C₁₂heterocycloalkyl; or
C₁-C₁₈alkyl, C₂-C₁₈alkenyl, C₂-C₁₈ alkynyl, C₇-C₉phenylalkyl, C₃-C₁₂cycloalkyl or C₂-C₁₂heterocycloalkyl, which are substituted by NO₂, halogen, amino, hydroxy, cyano, carboxy, C₁-C₄alkoxy, C₁-C₄alkylthio, C₁-C₄alkylamino or di(C₁-C₄alkyl)amino; or
phenyl, naphthyl, phenanthryl, anthryl, 5,6,7,8-tetrahydro-2-naphthyl, pyrrolyl, imidazolyl, pyrazolyl, pyrazinyl, pyrimidinyl, pyridazinyl, indolizinyl, isoindolyl, indolyl, indazolyl, purinyl, quinolizinyl, isoquinolyl, quinolyl, quinoxalinyl, quinazolinyl, cinnolinyl, pteridinyl, phenazinyl, isothiazolyl, phenothiazinyl, isoxazolyl, furazanyl, biphenyle or phenoxazinyl, which are unsubstituted or substituted by C₁-C₄alkyl, C₁-C₄alkoxy, C₁-C₄alkylthio, halogen, cyano, hydroxy, carboxy, C₁-C₄alkylamino or di(C₁-C₄alkyl)amino;
or R₂ and R₃, together with the linking carbon atom, form a C₃-C₁₂cycloalkyl radical, a (C₄-C₁₂cycloalkanon)-yl radical or a C₂-C₁₂heterocycloalkyl radical with at least one O atom and/or a NR₈ group;
R₄ is C₁-C₁₈alkyl, phenyl, an alkali metal cation or a tetraalkylammonium cation;
R₅ and R₆ are hydrogen, C₁-C₁₈alkyl, C₂-C₁₈alkyl which is substituted by at least one hydroxy group or, taken together, form a C₂-C₁₂alkylene bridge or a C₂-C₁₂-alkylene bridge interrupted by at least one O or/and NR₈ atom;
R₇ is hydrogen, C₁-C₁₈alkyl or phenyl;
R₈ is C₁-C₁₈alkyl or C₂-C₁₈alkyl which is substituted by at least one hydroxy group;
R₉ is C₁-C₄alkylen or a direct bond;
or R₁, R₂ and R₃ form together the residue of a polycyclic cycloaliphatic ring system or a polycyclic heterocycloaliphatic ring system with at least one di-or trivalent nitrogen atom and
with the proviso that,
if R₂ and R₃ are methyl, R₁ is not methyl, hydroxymethyl, -C(O)O-CH₃, -C(O)O-C₂H₅, NO₂ or CN;
if R₂ and R₃, together with the linking carbon atom, form a C₅ or C₆cycloalkyl radical, R₁ is not -CN or -C(O)OCH₃
if R₂ and R₃ together are cyclopentanone, R₁ is not methyl and
R₁, R₂ and R₃ are not F at the same time.

21. A process for preparing a compound of formula (I) as defined in claim 1 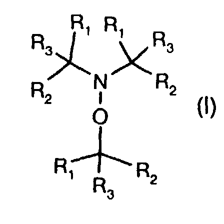 by generating a free radical ·CR₁R₂R₃
from a compound capable of eliminating a neutral molecule, or undergoing C-C bond-scission upon thermal or photochemical treatment, or by hydrogen abstraction from a compound R₁R₂R₃C-H in reaction with reactive radicals, and reacting the free radical ·CR₁R₂R₃ with NO in a solvent which does not interfere with the radical reaction.

22. A process according to claim 21 wherein the free radical ·CR₁R₂R₃ is prepared by heating or irradiating a compound of formula IIIa, IIIb or IIIc

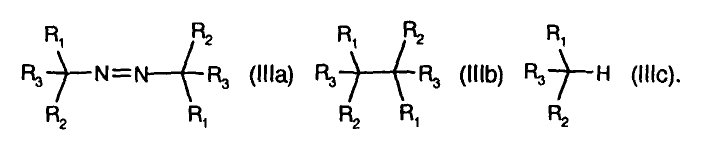

23. A process according to claim 21, wherein the radical ·CR₁R₂R₃ is prepared by a thermal reaction at a temperature from 20 to 150° C.

24. A process according to claim 21, wherein the free radical source is 2,2'-azobisisobutyronitrile, 2,2'-azobis(2-methylbutyronitrile), 2,2'-azobis(2,4-dimethylvaleronitrile), 2,2'-azobis(4-methoxy-2,4-dimethylvaleronitrile), 1,1'-azobis(1-cyclohexanecarbonitrile), 4,4'-azobis(4-cyanopentanoic acid), 2,2'-azobis(isobutyramide), dihydrate, 2-phenylazo-2,4-dimethyl-4-methoxyvaleronitrile, dimethyl-2,2'-azobisisobutyrate, 2-(carbamoylazo)isobutyronitrile, 2,2'-azobis(2,4,4-trimethylpentane), 2,2'-azobis(2-methylpropane), 2,2'-azobis(N,N'-dimethyleneisobutyramidine), free base or hydrochloride, 2,2'-azobis(2-amidinopropane), free base or hydrochloride, 2,2'-azobis{2-methyl-N-[1,1-bis(hydroxymethyl)ethyl]propionamide} or 2,2'-azobis{2-methyl-N-[1,1-bis(hydroxymethyl)-2-hydroxyethyl]propionamide.

25. Use of a compound of formula I according to claim 1 for polymerizing ethylenically unsaturated monomers.

26. A polymerizable composition, comprising
a) at least one ethylenically unsaturated monomer or oligomer,
b) a compound of formula (IV) and c) a radical iniator according to claim 21 capable of generating a free radical of formula (V) 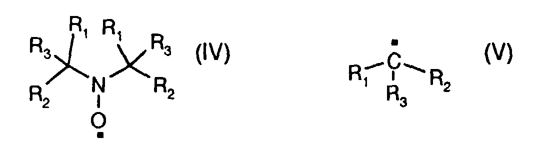
wherein R₁, R₂, R₃ are each independently of one another hydrogen, halogen, NO₂, cyano, -CONR₅R₆, -(R₉)COOR4, -C(O)-R₇, -OR₈, -SR₈, -NHR₈, -N(R₈)₂, carbamoyl, di(C₁-C₁₈alkyl)carbamoyl, -C(=NR₅)(NHR₆);
unsubstituted C₁-C₁₈alkyl, C₂-C₁₈alkenyl, C₂-C₁₈alkynyl, C₇-C₉phenylalkyl, C₃-C₁₂cycloalkyl or C₂-C₁₂heterocycloalkyl; or
C₁-C₁₈alkyl, C₂-C₁₈alkenyl, C₂-C₁₈ alkynyl, C₇-C₉phenylalkyl, C₃-C₁₂cycloalkyl or C₂-C₁₂heterocycloalkyl, which are substituted by NO₂, halogen, amino, hydroxy, cyano, carboxy, C₁-C₄alkoxy, C₁-C₄alkylthio, C₁-C₄alkylamino or di(C₁-C₄alkyl)amino; or
phenyl, naphthyl, phenanthryl, anthryl, pyrrolyl, imidazolyl, pyrazolyl, pyrazinyl, pyrimidinyl, pyridazinyl, indolizinyl, isoindolyl, indolyl, indazolyl, purinyl, quinolizinyl, isoquinolyl, quinolyl, quinoxalinyl, quinazolinyl, cinnolinyl, pteridinyl, phenazinyl, isothiazolyl, phenothiazinyl, isoxazolyl, furazanyl, biphenyle or phenoxazinyl, which are unsubstituted or substituted by C₁-C₄alkyl, C₁-C₄alkoxy, C₁-C₄alkylthio, halogen, cyano, hydroxy, carboxy, C₁-C₄alkylamino or di(C₁-C₄alkyl)amino;
or R₂ and R₃, together with the linking carbon atom, form a C₃-C₁₂cycloalkyl radical, a (C₄-C₁₂cycloalkanon)-yl radical or a C₂-C₁₂heterocycloalkyl radical containing at least one O atom and/or a NR₈ group;
R₄ is hydrogen, C₁-C₁₈alkyl, phenyl, an alkali metal cation or a tetraalkylammonium cation;
R₅ and R₆ are hydrogen, C₁-C₁₈alkyl, C₂-C₁₈alkyl which is substituted by at least one hydroxy group or, taken together, form a C₂-C₁₂alkylene bridge or a C₂-C₁₂-alkylene bridge interrupted by at least one O or/and NR₈ atom;
R₇ is hydrogen, C₁-C₁₈alkyl or phenyl;
R₈ is hydrogen, C₁-C₁₈alkyl or C₂-C₁₈alkyl which is substituted by at least one hydroxy group;
R₉ is C₁-C₁₂alkylen;
or R₁, R₂ and R₃ form together the residue of a polycyclic cycloaliphatic ring system or a polycyclic heterocycloaliphatic ring system with at least one di-or trivalent nitrogen atom.

27. A process for preparing an oligomer, a cooligomer, a polymer or a copolymer (block or random) by free radical polymerization of at least one ethylenically unsaturated monomer/oligomer, which comprises subjecting a composition according to claim 26 to heat or actinic radiation.
